# EUROPEAN PATENT APPLICATION

(11) **EP 4 810 086 A1**
(43) Date of publication of application: **23.09.2026**
(21) Application number: 25305380.5
(22) Date of filing: 19.03.2025
(51) Int. Cl.: A61M 5/32, A61M 5/31

(54) **SYRINGE NEEDLE SHIELD WITH NON-CIRCULAR SEALING RING**

(71) Applicant: Becton Dickinson France, 38800 Le Pont-de-Claix (FR)
(72) Inventor: LEIBBRAND, Alfred, 38640 Claix (FR); PETIT, Louis, 38610 Venon (FR); TOURNAIRE, Maxime, 38100 Grenoble (FR); REMPFER, Simon, 38260 Saint-Hilaire-de-la-Côte (FR); DEVILLARD, Sylvain, 38000 Grenoble (FR); PILOY, Jefferson, 38000 Grenoble (FR)
(74) Representative: Germain Maureau

(57) **Abstract**

Provided herein is a needle shield for use with a syringe comprising a needle and a barrel. The needle shield includes a rigid casing portion and a compliant casing portion joined with the rigid casing portion and formed of an elastomeric material. The compliant casing portion has a closed distal end and an open proximal end and defining a cavity therein, with the hollow cavity comprising a needle storage portion within which the needle is positioned when the needle shield is mounted to the barrel and a barrel storage portion within which the hub portion of the barrel is received. The compliant casing portion comprises a sealing ring formed on an inner surface thereof, in an area of the barrel storage portion, and that engages the hub portion of the barrel when the needle shield is mounted to the barrel, the sealing ring having a non-circular shape.

## Description

### BACKGROUND OF THE INVENTION

### Field of the Invention

The present disclosure relates generally to syringes and, more particularly, to a needle shield for a syringe, with the needle shield comprising a barrel end storage portion that includes a non-circular or oval-shaped sealing ring configured to receive a syringe barrel hub therein, and with the non-circular or oval-shaped sealing ring reducing the force needed to remove the needle shield from the syringe barrel.

### Description of Related Art

Syringes are used in a variety of environments for administering fluids, such as medications or other drugs, to a patient. Many syringes are provided as prefilled syringes, which provide the convenience of rapidly delivering the liquid therein to a patient without the need to first aspirate the medication from another container and meter its volume. The prefilled syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

It is recognized that syringes must be handled with care before and after use due to the presence of the needle. To minimize the risk of accidental injury due to needle sticks, the syringe typically includes a needle shield that covers the sharpened tip of the needle. The needle shield is removed prior to use to expose the sharpened tip of the needle. Such a shield also serves to protect the sharpened tip of the needle and to preserve its sterility prior to use of the injection device.

Needle shields are generally formed to include a rigid component and a soft component. The soft component is typically formed of elastic rubber (e.g., isoprene rubber, butyl rubber, latex rubber, silicone rubber or the like) of a thermoplastic elastomer (TPE) that provides a secure sealing connection with the syringe, at least along a sealing line, and the rigid component provides protection against accidental needle sticks and provides the user with a grippable surface for the user to remove the needle shield from the syringe.

With syringes, it is desirable for the needle shield to be removable in a predictable and consistent manner. That is, with existing syringes, it is recognized that the construction of the needle shield can contribute to the pull-out force required to remove the needle shield. As examples, the formulation of the soft component of the needle shield and/or the amount and location of contact between the soft component and the syringe barrel can contribute to the amount of pull-out force required to remove the needle shield.

With regard to the amount and location of contact between the soft component and the syringe barrel contributing to the amount of pull-out force required to remove the needle shield, it is recognized that many existing needle shields can include a soft component having a circular sealing ring used to secure the needle shield to a syringe barrel hub. Such a circular sealing ring deforms symmetrically when the needle shield is removed from the syringe barrel hub, which leads to a symmetry of forces when pulling off the needle shield and releasing/breaking the covalent bonds between the sealing ring and the hub, leading to an increase in the pull-out force required to remove the needle shield.

Accordingly, a need exists in the art for a needle shield that exhibits a predictable and consistent pull-out force for removal thereof from the syringe. It is desirable for the needle shield to include a soft component with a sealing ring that provides asymmetrical forces when removing the needle shield, so as to reduce the pull-out force.

### SUMMARY OF THE INVENTION

Provided herein is a needle shield for use with a syringe comprising a needle and a barrel. The needle shield includes a compliant casing portion having a closed distal end and an open proximal end and defining a cavity therein, with the hollow cavity comprising a needle storage portion within which the needle is positioned when the needle shield is mounted to the barrel and a barrel storage portion within which the hub portion of the barrel is received. The compliant casing portion comprises a sealing ring formed on an inner surface thereof, in an area of the barrel storage portion, and that engages the hub portion of the barrel when the needle shield is mounted to the barrel, the sealing ring having a non-circular shape.

In accordance with some aspects of the disclosure, the sealing ring comprises an oval-shaped sealing ring.

In accordance with some aspects of the disclosure, opposing sides or ends of the oval-shaped sealing ring are positioned at different longitudinal locations on the needle shield, such that the oval-shaped sealing ring has a measurable amplitude in a longitudinal direction of the needle shield.

In accordance with some aspects of the disclosure, the amplitude of the oval-shaped sealing ring is less than a length of the hub portion in the longitudinal direction.

In accordance with some aspects of the disclosure, an increase of the amplitude of the oval-shaped sealing ring increases the ovalization of the sealing ring, with the ovalization of the sealing ring proportional to a shear stress present at the sealing ring when the needle shield is removed from the hub portion.

In accordance with some aspects of the disclosure, the sealing ring comprises one or more ribs each protruding radially inward from the inner surface, with each of the one or more ribs comprises an oval-shaped rib.

In accordance with some aspects of the disclosure, the one or more ribs comprises a pair of ribs spaced apart in the longitudinal direction.

In accordance with some aspects of the disclosure, each of the one or more ribs is formed to have a high rounded edge profile, a low rounded edge profile, a knife edge profile, or a square edge profile.

In accordance with some aspects of the disclosure, the oval-shaped sealing ring has a smooth inner contact surface, with a period of the oval-shaped sealing ring being 360°.

In accordance with some aspects of the disclosure, the oval-shaped sealing ring has a contoured inner contact surface, with a period of the oval-shaped sealing ring being less than 360°.

In accordance with some aspects of the disclosure, the sealing ring has a contact surface having a sinusoidal or wavy configuration.

In accordance with some aspects of the disclosure, the non-circular shape of the sealing ring is configured to provide an asymmetry of forces when pulling off the needle shield from the hub portion and breaking covalent bonds between the sealing ring and the hub portion, thereby decreasing a pull-out force required to remove the needle shield from the hub portion.

In accordance with some aspects of the disclosure, the compliant casing portion is formed of an elastomeric material comprising an elastic rubber or a thermoplastic elastomer.

In accordance with some aspects of the disclosure, the needle shield further comprises a rigid casing portion, wherein the rigid casing portion comprises one of a rigid outer casing forming at least a portion of an outer surface of the needle shield, and wherein the compliant casing portion comprises a compliant inner casing positioned within the rigid outer casing, or a rigid core embedded within the compliant casing portion, and wherein the compliant casing portion comprises a compliant skin layer formed about the rigid core.

Also provided herein is a prefilled or prefillable syringe that comprises a syringe assembly including a barrel having a barrel distal end and a barrel proximal end and defining a chamber, the barrel including a barrel end portion at the barrel distal end, a needle having a needle tip at a distal end of the needle and having a proximal end fixedly inserted into the barrel end portion, and a needle shield, with the needle shield mounted to the barrel, so as to be positioned over the needle. The needle shield includes a rigid casing portion and a compliant casing portion joined with the rigid casing portion and formed of an elastomeric material. The compliant casing portion has a closed distal end and an open proximal end and defining a cavity therein, with the hollow cavity comprising a needle storage portion within which the needle is positioned when the needle shield is mounted to the barrel and a barrel storage portion within which the hub portion of the barrel is received. The compliant casing portion comprises a sealing ring formed on an inner surface thereof, in an area of the barrel storage portion, and that engages the hub portion of the barrel when the needle shield is mounted to the barrel, the sealing ring having a non-circular shape. The prefilled or prefillable syringe also includes a plunger received within the chamber of the barrel and axially movable therein.

### BRIEF DESCRIPTION OF THE DRAWINGS

FIG. 1 is a perspective view of a syringe including a needle shield, according to an embodiment of the disclosure;
FIG. 2 is an exploded view of the syringe of FIG. 1;
FIG. 3 is a cross-sectional perspective view taken along line 3-3 of FIG. 1, showing the needle shield secured on the hub portion of the syringe barrel, according to an embodiment of the disclosure;
FIG. 4 is a cross-sectional side view of the compliant inner casing of the needle shield of FIG. 3 shown in isolation;
FIG. 5 is a cross-sectional perspective view taken along line 3-3 of FIG. 1, showing the needle shield secured on the hub portion of the syringe barrel, according to another embodiment of the disclosure;
FIG. 6A is a detailed profile view of a sealing ring rib of the compliant inner casing of FIG.4, according to an embodiment of the disclosure;
FIG. 6B is a detailed profile view of a sealing ring rib of the compliant inner casing of FIG.4, according to an embodiment of the disclosure;
FIG. 6C is a detailed profile view of a sealing ring rib of the compliant inner casing of FIG.4, according to an embodiment of the disclosure;
FIG. 6D is a detailed profile view of a sealing ring rib of the compliant inner casing of FIG.4, according to an embodiment of the disclosure; and
FIG. 7 is a cross-sectional view of a needle shield, according to another embodiment of the disclosure.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

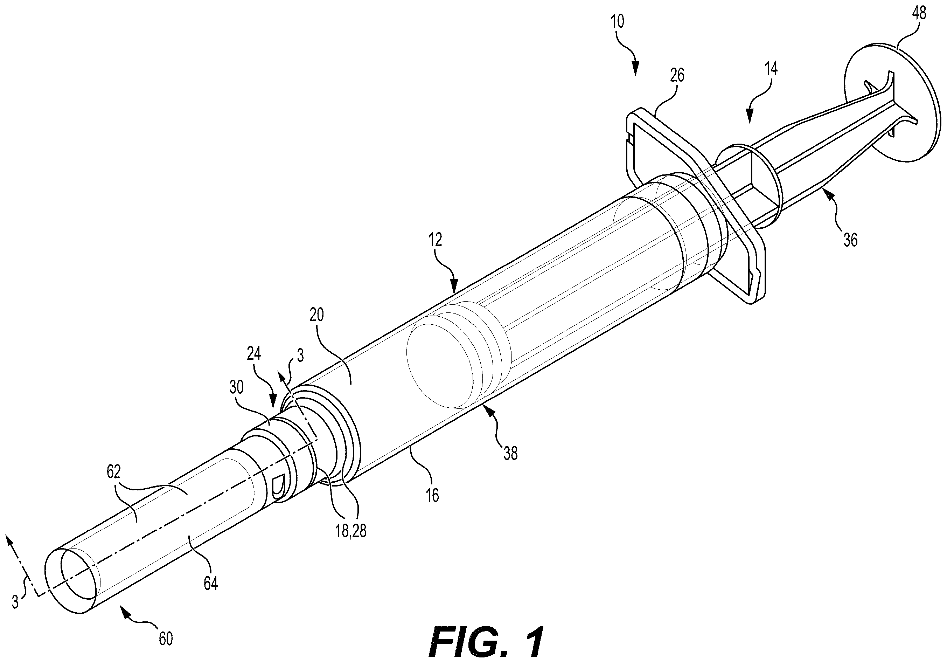

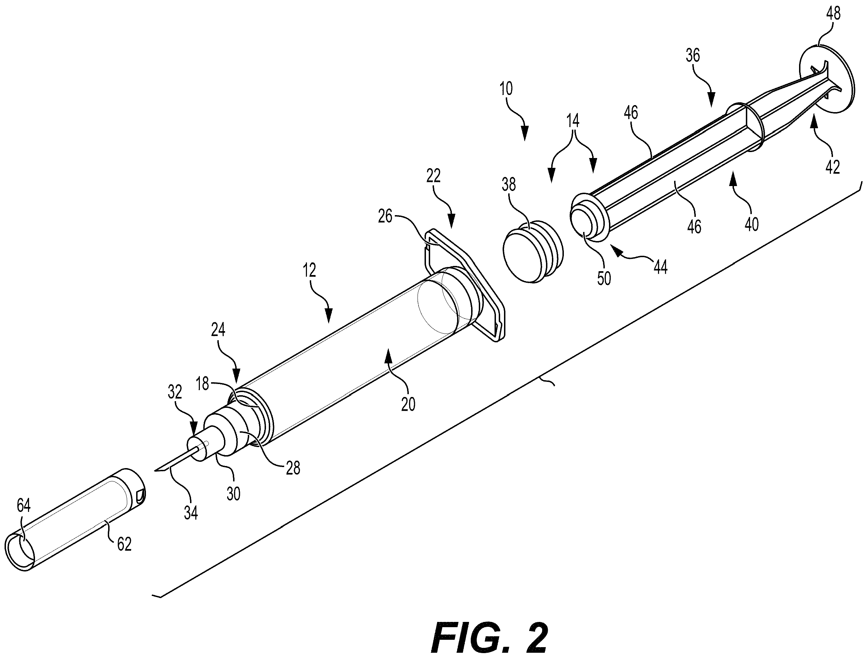

Referring to FIGS. 1 and 2, shown is a non-limiting embodiment of a medical injection device 10, such as a syringe for example, with which aspects or embodiments of the disclosure may be implemented. While the medical injection device is shown and described hereafter as a syringe 10, it is recognized that other medical injection devices may incorporate aspects of the disclosure, as described in detail here below.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the tip member 18 may include a hub portion 30 that narrows with respect to the cylindrical outer wall 16 and that is formed as a partially hollow member that defines a channel 32 therethrough in fluid communication with the chamber 20. A needle 34 is attached to the hub portion 30 within channel 32, with a proximal end of the needle 34 positioned within the channel 32 and a distal end of the needle 34 extending out from the hub portion 30. The proximal end of the needle 34 may be inserted into the channel 32 of hub portion 30 and may be fixed therein with an adhesive (e.g., glue), by thermal welding, or the like. The needle 34 defines a hollow lumen therein that is in fluid communication with the chamber 20 of syringe barrel 12, such that fluid may be dispensed from the syringe 10. In some embodiments, the tip member 18 may also include a shoulder 28 positioned between the cylindrical outer wall 16 and the hub portion 30, with the shoulder 28 having an intermediate diameter that provides a transition between the cylindrical outer wall 16 and the hub portion 30 - but in other embodiments the shoulder 28 may be omitted.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. In some embodiments, a flanged extension member 50 (e.g., disc-shaped flange) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper, with the protrusion and receptacle engaging via threading, for example.

The stopper 38 of plunger assembly 14 is positioned at the plunger distal end 44 so as to be movable along with the plunger 36 within the chamber 20 of syringe barrel 12. The stopper 38 may be made from a material that is different from the material of the plunger 36 and that is capable of forming a tight seal with the syringe barrel 12 as it is advanced therethrough. In some embodiments, the stopper 38 includes a receptacle (not shown) formed therein that is sized and configured to receive the flanged extension member 50 of plunger 36, with the flanged extension member 50 coupling with the receptacle via a press fit connection, for example, to secure the stopper 38 to the plunger 36, although it can be appreciated that the stopper 38 and plunger distal end 44 can be secured by other techniques known in the art.

As shown in FIGS. 1 and 2, a needle shield 60 of syringe assembly 12 is coupled to the syringe barrel 12 to protect the needle 34. According to aspects of the disclosure, the needle shield 60 is configured to be mounted to the barrel 12 via coupling of the needle shield 60 to the hub portion 30 of the barrel 16. As explained in further detail below, the needle shield 60 may comprise a rigid casing portion 62 composed of a rigid material or semi-rigid material that provides structure to the needle shield 60, as well as a compliant casing portion 64 composed of an elastomeric material (i.e., a deformable or compliant material) that surrounds the needle 34 when the needle shield 60 is coupled to the syringe barrel 12 and may also present a mating surface for the hub portion 30 of barrel 12. In various embodiments, the rigid material may be a resin such as polypropylene (PP), polyethylene (PE), polyethylene terephthalate (PET), polystyrene (PS), polycarbonate (PC), or polyvinyl chloride (PVC), as non-limiting examples, while the elastomeric material may be an elastic rubber (e.g., isoprene rubber, butyl rubber, latex rubber, silicone rubber or the like) or a thermoplastic elastomer (TPE), as non-limiting examples.

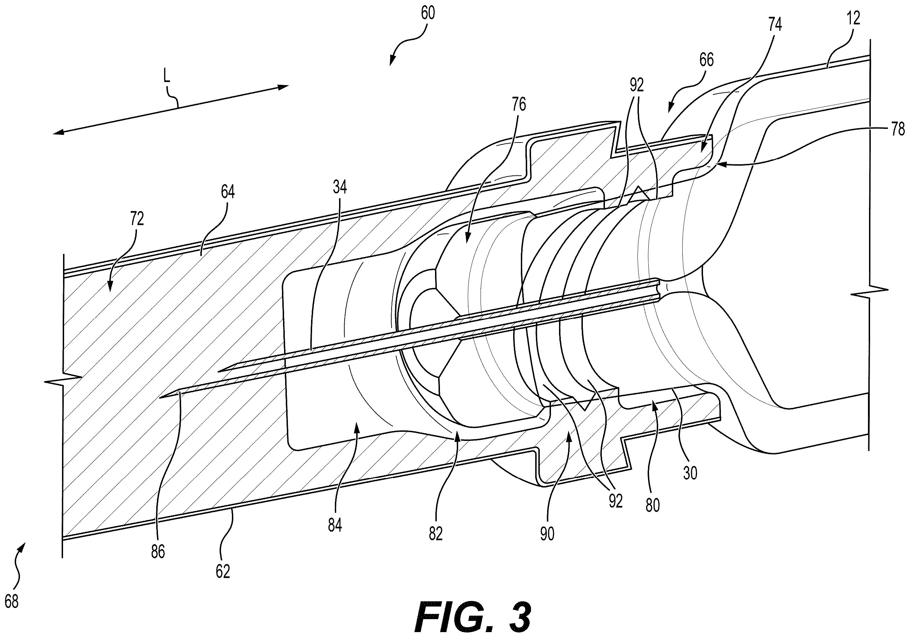

As shown best in FIG. 3, according to an exemplary embodiment of the disclosure, the rigid casing portion 62 may comprise a rigid outer casing (hereafter "rigid outer casing 62" that provides structure to the needle shield 60 and the compliant casing portion 64 may comprise a compliant inner casing 64 that surrounds the needle 34 (when needle shield 60 is coupled to syringe barrel 12).

The rigid outer casing 62 may be constructed as a generally tubular member that includes an open proximal end portion 66 and a mostly closed distal end portion 68. The distal end portion 68 may include an opening 70 therein having a smaller diameter than a diameter of the compliant inner casing 64, such that the compliant inner casing 64 may be better retained in the rigid outer casing 62. The compliant inner casing 64 is formed as an elongated cylindrical body that is sized to be positioned within the outer casing 62, so as to be retained therein. The compliant inner casing 64 may be characterized as having a closed distal end portion 72 and an open proximal end portion 74. A hollow cavity 76 is formed in the compliant inner casing 64, with an opening 78 in the compliant inner casing 64 providing access into the cavity 76. The cavity 76 extends distally into the compliant inner casing 64 a portion of the length thereof.

According to aspects of the disclosure, the hollow cavity 76 may be characterized as including a barrel end storage portion 80 and a needle storage portion 82. The open proximal end 74 (i.e., opening 80 therein) allows for positioning of the needle shield 60 onto the hub portion 30 of syringe barrel 12. The hub portion 30 may be introduced through open proximal end 74 and positioned within the barrel end storage portion 80, with the hub portion 30 sized to form a tight fit within the barrel end storage portion 80 to ensure engagement of the needle shield 60 to the barrel 12.

The needle storage portion 82 is sized to receive the needle 34 therein, to provide protection to the needle 34 once the needle shield 60 is coupled to barrel 12. According to embodiments of the disclosure, the needle storage portion 82 has an inner diameter that tapers as the needle storage portion 82 extends from its proximal end to its distal end. The distal end of the needle storage portion 82 is formed with a small diameter tip section 84 within which a distal end of the needle 34 may be received.

According to some embodiments of the disclosure, the hollow cavity 76 of needle shield 60 may be configured such that, when the hub portion 30 of the barrel 12 is inserted into the needle shield 60 and is received in the barrel end storage portion 80 of the needle shield 60, a sharpened tip 86 of the needle 34 enters the small diameter tip section 84 of the needle storage portion 82 and protrudes therethrough to reach the compliant material of the closed distal end 72 of inner casing 64 - i.e., the needle tip 86 punctures and extends into the compliant material - such that the needle tip 86 is sealed in the compliant material of the inner casing 64. With the needle tip 86 sealed in the compliant material of the inner casing 64, leakage of fluid out from the needle 34 may be prevented. In other embodiments, the needle storage portion 82 may be configured such that the sharpened tip 86 of the needle 34 does not protrude through the small diameter tip section 84 so as to reach the compliant material of closed distal end 72 - i.e., the sharpened tip 86 does not puncture and extend into compliant inner casing 64.

According to aspects of the disclosure, the compliant inner casing 64 may be specifically configured such that the barrel end storage portion 80 defined thereby receives and engages with the hub portion 30 of barrel 12 in such a manner that reduces the pull-out force required to remove the needle shield 60 from the syringe barrel 12. In particular, the compliant inner casing 64 includes an inner sealing ring 90 formed thereon, adjacent the open proximal end portion 74 thereof and in the barrel end storage portion 80 of cavity. The sealing ring 90 extends radially inward from a remainder of the inner surface 92 of the compliant inner casing 64 at the proximal end portion 74, to provide contact and sealing between the compliant inner casing 64 and the hub portion 30 of barrel 12. In some embodiments, the sealing ring 90 may be formed by a pair of ribs 92 spaced apart in a longitudinal direction L of the needle shield 60. In other embodiments, it is recognized that the sealing ring 90 could instead be formed by a single rib 92 or by more than two ribs 92 (e.g., three ribs 92) spaced apart in the longitudinal direction L. When discussing the configuration of the sealing ring 90 hereafter, it is recognized that such discussion is also directed to the configuration of each of the rib(s) 92 forming the sealing ring 90.

According to embodiments, the sealing ring 90 is designed to provide an asymmetry of forces when pulling off the needle shield 60 from the hub portion 30 and releasing/breaking the covalent bonds between the sealing ring 90 and the hub portion 30 that hold the needle shield 60 on the hub portion 30, thereby decreasing the pull-out force required to remove the needle shield 60 from the hub portion 30. That is, the shape of the sealing ring 90 creates singular areas of pressure concentration that allows for breaking of the covalent bonds using less user-applied force (i.e., pressure is the ratio of force divided by surface (contact), thus by reducing the surface contact, a given pressure is achieved using less force and applied in a specific area).

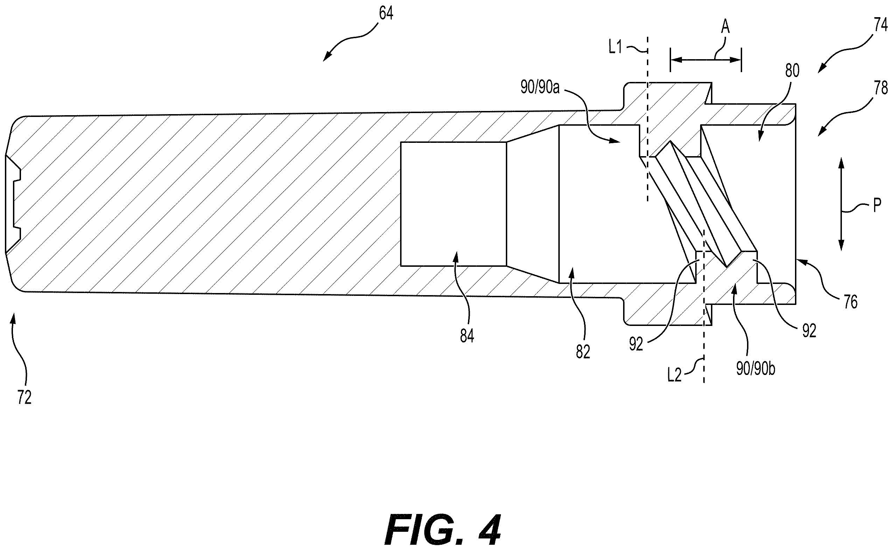

Referring to FIGS. 3 and 4, it is seen that the sealing ring 90 is configured as a non-circular or "oval" shaped sealing ring 90 having opposing sides 90a, 90b that are positioned at different longitudinal locations L1, L2 on the needle shield 60. That is, instead of the sealing ring 90 being formed as a circular sealing ring 90 formed along a cross-section of the needle shield 60, at a singular longitudinal point/location on the needle shield 60, the oval-shaped sealing ring 90 instead is formed at an angle, such that the oval-shaped sealing ring 90 has a measurable length or "amplitude" A in the longitudinal direction L - i.e., a first side or point 90a on the oval-shaped sealing ring 90 is formed on compliant inner casing 64 at a first longitudinal location L1 and an opposing second side or point 90b on the oval-shaped sealing ring 90 is formed on compliant inner casing 64 at a second longitudinal location L2 that is spaced apart from the first longitudinal location L1. According to aspects of the disclosure, the amplitude A of the oval-shaped sealing ring 90 can vary but must be less than a length L_{H} of the hub portion 30 of the barrel 12, such that opposing sides 90a, 90b of the sealing ring 90 (at different longitudinal locations L1, L2) will both be in contact with the hub portion 30, and such that the sealing ring 90 is properly secured/sealed onto the hub portion 30.

According to aspects of the disclosure, the length/amplitude A of the oval-shaped sealing ring 90 will impact the non-circularity (i.e., ovalization) of the sealing ring 90 and thus also impact the shear-stress-increasing properties of the sealing ring 90 when the needle shield 60 is removed from the hub portion 30. That is, it is recognized that with the sealing ring 90 being formed with a non-circular or oval shape, the compliant material of the sealing ring 90 will deform asymmetrically as the needle shield 60 is pulled off of the hub portion 30, which causes a shear stress to locally increase at points on the sealing ring 90, thereby allowing covalent bonds between the sealing ring 90 and the hub portion 30 to be released/broken asymmetrically and with reduced force (i.e., reduced pull-out force). The amplitude A of the sealing ring 90 impacts the ovalization and the shear-stress-increasing properties of the sealing ring 90, with the larger the amplitude A of the sealing ring 90 corresponding to the larger the local increase of shear stress at points on the sealing ring 90, and with it recognized that the amplitude A of the sealing ring 90 is adapted to other needle shield parameters, such as a shore hardness of the compliant material from which compliant inner casing 64 is formed, in order to maintain functionality of the needle shield 60, including proper sealing between the compliant inner casing 64 and the hub portion 30.

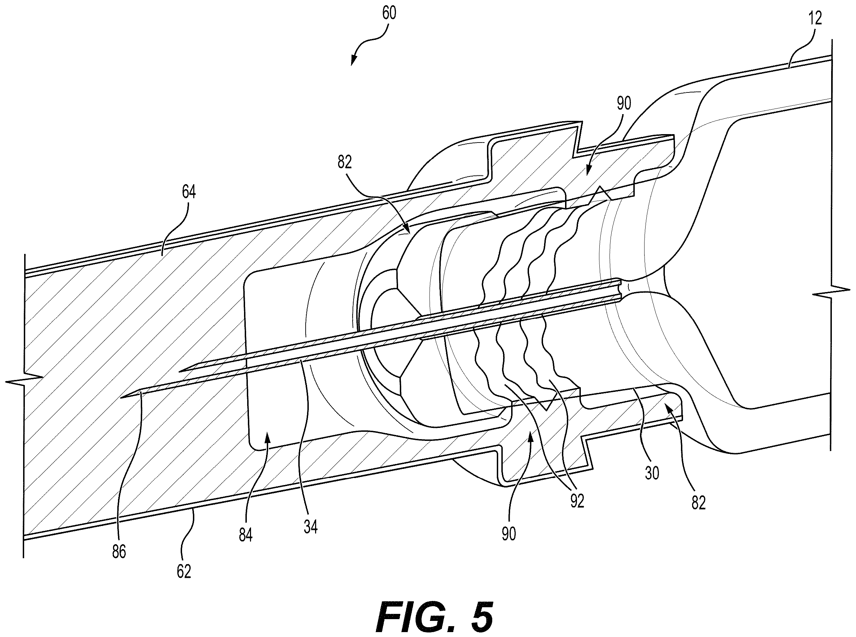

According to some embodiments of the disclosure, in addition to the sealing ring 90 being configured as an oval-shaped sealing ring 90 having an amplitude A along a longitudinal direction L of the compliant inner casing 64, the sealing ring 90 may also be configured to have a period P that may be varied/changed to alter the symmetry of forces when pulling the needle shield 60 off of the hub portion 30. That is, when the sealing ring 90 is formed as a simple oval (having a smooth inner contact surface 94), the sealing ring 90 is considered to have a 360° period. However, it is recognized that the period P of the sealing ring 90 could be increased (to less than 360°) to alter the symmetry of forces, such as by forming the sealing ring 90 to have an inner edge/surface having a sinusoidal or "wavy" configuration (i.e., each rib 92 of the sealing ring 90 has a sinusoidal or wavy configuration), as shown in FIG. 5. With the sealing ring 90 having such a wavy configuration, the number of "high points" or "contact points" where detachment of the sealing ring 90 from the hub portion 30 initiates is increased - thereby impacting the shear-stress-increasing properties of the sealing ring 90, with the larger the number of high points of the sealing ring 90 corresponding to the larger the local increase of shear stress at points on the sealing ring 90.

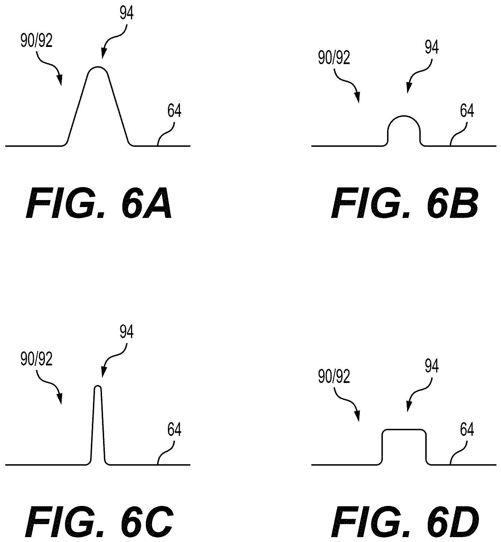

According to still further aspects of the disclosure, it is recognized that a shape/configuration of the contact surface 94 of the sealing ring 90 (i.e., the surface of sealing ring 90, and each rib 92 thereof, in contact with hub portion 30) may be selected to further control sealing between the compliant inner casing 64 and hub portion 30, including optimizing the pull-out force of the needle shield 60. That is, a depth, width, and/or shape of the sealing ring 90 and sealing ring contact surface 94 may be selected/controlled in order to optimize the pull-out force of the needle shield 60, with the specific configuration of the contact surface 94 affecting a profile/shape of the contact interface where detachment of the sealing ring 90 from the hub portion 30 initiates - thereby impacting the shear-stress-increasing properties of the sealing ring 90. Exemplary designs are illustrated in FIGS. 6A-6D and may be characterized as a high rounded edge profile (FIG. 6A), a low rounded edge profile (FIG. 6B), a knife edge profile (FIG. 6C), and a square edge profile (FIG. 6D). It is recognized that the design of the sealing ring 90 is not meant to be limited to the specific designs shown/described here, and that other suitable designs are envisioned, limited only by the sealing ring 90 being compatible with current rubber manufacturing techniques, such as compression and injection molding and fast de-molding of the compliant inner casing 64 that does not damage the sealing ring 90.

Based on the construction of the inner casing 64 described above, including the configuration of sealing ring 90 for engagement of the needle shield 60 to the hub portion 30 as described above, the pull-out force required for removal of the needle shield 60 can be kept low. In removing the needle shield 60 from the hub portion 30 of syringe barrel 12, a distally directed axial pulling force may be applied to the needle shield 60, such as by a healthcare provider grasping the outer casing 62 and pulling distally on the outer casing 62. With the sealing ring 90 having an oval shape, the compliant material of the sealing ring 90 will deform asymmetrically as the needle shield 60 is pulled off of the hub portion 30, which causes a shear stress to locally increase at points on the sealing ring 90, thereby allowing covalent bonds between the sealing ring 90 and the hub portion 30 to be released/broken asymmetrically and with reduced force (i.e., reduced pull-out force), thereby decreasing the pull-out force required to remove the needle shield 60 from the hub portion 30.

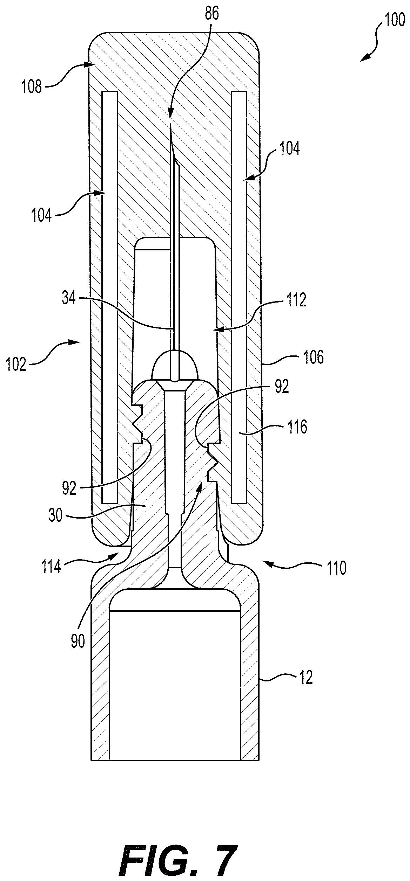

While the needle shield 60 shown and described in FIGS. 1-6 is characterized as including a rigid outer shell 62 and a compliant inner shell 64, it is recognized that embodiments of the disclosure also encompass needle shields with other constructions. For example, according to another embodiment shown in FIG. 7, a needle shield 100 may be provided that includes a compliant casing portion in the form of a compliant skin layer (hereafter "compliant skin layer 102") and a rigid casing portion 104 in the form of a rigid core (hereafter "rigid core 104"), with the compliant skin layer 102 formed of an elastomeric material and the rigid core 104 formed of a rigid material. As previously described, the rigid material may be polypropylene (PP), polyethylene (PE), polyethylene terephthalate (PET), polystyrene (PS) or polycarbonate (PC), as non-limiting examples, while the elastomeric material may be an elastic rubber or thermoplastic elastomer.

As shown in FIG. 7, the skin layer 102 may be generally structured as a cylindrical body 106 having a closed distal end 108 and an open proximal end 110. A hollow cavity 112 is formed in the cylindrical body 106 at the open proximal end 110 thereof, with an opening 114 in the cylindrical body 106 providing access into the cavity 112. The cavity 112 extends distally into the cylindrical body 106 a portion of the length of the cylindrical body 106. The cavity 112 is sized and positioned to receive at least a portion of the needle 34 therein, to provide protection to the needle 34 once the needle shield 100 is coupled to the syringe barrel 12. In some embodiments, the cavity 112 is configured such that, when the needle shield 100 is secured on syringe barrel 12, the needle 34 extends through a length of the cavity 112, such that a needle tip 86 protrudes through the cavity 112 to reach the elastomeric material of closed distal end 108 that is positioned distally from cavity 112 - i.e., the needle tip 86 punctures and extends into the elastomeric material - such that the needle tip 86 is sealed in the elastomeric material of the skin layer 102. With the needle tip 86 sealed in the elastomeric material of the skin layer 102, leakage of fluid out from the needle 34 may be prevented.

As shown in FIG. 7, the core 104 is constructed as a tubular member 116 that is embedded within the skin layer 102. The core 104 may extend a majority of a length of the overall needle shield 100, from a location adjacent the closed distal end 108 to a location adjacent the open proximal end 110. The core 104 functions to add stiffness to the overall needle shield 100 - preventing an excessive amount of bending, flexing or overall deformation of the skin layer 102.

Similar to the needle shield 60 of FIGS. 1-6, the compliant skin layer 102 is configured to include a sealing ring 90 (formed of rib(s) 92) thereon adjacent the open proximal end 110 and that functions to secure the needle shield 100 to the hub portion 30 of syringe barrel 12. As previously described, the sealing ring 90 is configured as a non-circular or "oval" shaped sealing ring 90 having opposing sides 90a, 90b that are positioned at different longitudinal locations L1, L2 on the needle shield 100. That is, instead of the sealing ring 90 being formed as a circular sealing ring 90 formed along a cross-section of the needle shield 100, at a singular longitudinal point/location on the needle shield 100, the oval-shaped sealing ring 90 instead is formed at an angle, such that the oval-shaped sealing ring 90 has a measurable length or "amplitude" A in the longitudinal direction L - i.e., a first side or point 90a on the oval-shaped sealing ring 90 is formed on compliant skin layer 102 at a first longitudinal location L1 and an opposing second side or point 90b on the oval-shaped sealing ring 90 is formed on compliant skin layer 102 at a second longitudinal location L2 that is spaced apart from the first longitudinal location L1. With the sealing ring 90 being formed with a non-circular or oval shape, the compliant material of the sealing ring 90 will deform asymmetrically as the needle shield 100 is pulled off of the hub portion 30, which causes a shear stress to locally increase at points on the sealing ring 90, thereby allowing covalent bonds between the sealing ring 90 and the hub portion 30 to be released/broken asymmetrically and with reduced force (i.e., reduced pull-out force).

In still other embodiments, it is recognized that other needle shields could be provided that include only a compliant portion (i.e., with no rigid cover or inserts). Similar to the needle shields 60, 100 previously shown and described in FIGS. 1-7, the compliant portion would include a sealing ring having ribs that function to secure the needle shield 1to the hub of the syringe barrel. As previously described, the sealing ring would be configured as a non-circular or "oval" shaped sealing ring having opposing sides that are positioned at different longitudinal locations on the needle shield, such that the oval-shaped sealing ring has a measurable length or "amplitude" in the longitudinal direction. With the sealing ring being formed with such a non-circular or oval shape, the compliant material of the sealing ring will deform asymmetrically as the needle shield is pulled off of the hub, which causes a shear stress to locally increase at points on the sealing ring, thereby allowing covalent bonds between the sealing ring and the hub to be released/broken asymmetrically and with reduced force (i.e., reduced pull-out force).

Beneficially, embodiments of the disclosure provide a needle shield that reduces the needle pull-out force required to remove the needle shield from the syringe barrel. The needle shield includes a shaped sealing ring by which a compliant inner casing is secured to a hub portion of the syringe barrel, with the sealing ring having an oval shape that functions to cause the sealing ring to deform asymmetrically as the needle shield is pulled off of the hub portion, which causes a shear stress to locally increase at points on the sealing ring, thereby allowing covalent bonds between the sealing ring and the hub portion to be released/broken asymmetrically and with reduced force, thereby decreasing the pull-out force required to remove the needle shield from the hub portion.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A needle shield for use with a syringe comprising a needle and a barrel, the needle shield comprising:
a compliant casing portion formed of an elastomeric material, the compliant casing portion having a closed distal end and an open proximal end and defining a cavity therein, the hollow cavity comprising a needle storage portion within which the needle is positioned when the needle shield is mounted to the barrel, and a barrel storage portion within which the hub portion of the barrel is received;
wherein the compliant casing portion comprises a sealing ring formed on an inner surface thereof, in an area of the barrel storage portion, and that engages the hub portion of the barrel when the needle shield is mounted to the barrel, the sealing ring having a non-circular shape.

2. The needle shield of claim 1, wherein the sealing ring comprises an oval-shaped sealing ring.

3. The needle shield of claim 2, wherein opposing sides or ends of the oval-shaped sealing ring are positioned at different longitudinal locations on the needle shield, such that the oval-shaped sealing ring has a measurable amplitude in a longitudinal direction of the needle shield.

4. The needle shield of claim 3, wherein the amplitude of the oval-shaped sealing ring is less than a length of the hub portion in the longitudinal direction.

5. The needle shield of claim 3, wherein an increase of the amplitude of the oval-shaped sealing ring increases the ovalization of the sealing ring, with the ovalization of the sealing ring proportional to a shear stress present at the sealing ring when the needle shield is removed from the hub portion.

6. The needle shield of claim 3, wherein the sealing ring comprises one or more ribs each protruding radially inward from the inner surface, with each of the one or more ribs comprises an oval-shaped rib.

7. The needle shield of claim 6, wherein the one or more ribs comprises a pair of ribs spaced apart in the longitudinal direction.

8. The needle shield of claim 6, wherein each of the one or more ribs is formed to have a high rounded edge profile, a low rounded edge profile, a knife edge profile, or a square edge profile.

9. The needle shield of claim 2, wherein the oval-shaped sealing ring has a smooth inner contact surface, with a period of the oval-shaped sealing ring being 360°.

10. The needle shield of claim 2, wherein the oval-shaped sealing ring has a contoured inner contact surface, with a period of the oval-shaped sealing ring being less than than 360°.

11. The needle shield of claim 10, wherein the sealing ring has a contact surface having a sinusoidal or wavy configuration.

12. The needle shield of claim 1, wherein the non-circular shape of the sealing ring is configured to provide an asymmetry of forces when pulling off the needle shield from the hub portion and breaking covalent bonds between the sealing ring and the hub portion, thereby decreasing a pull-out force required to remove the needle shield from the hub portion.

13. The needle shield of claim 1, wherein the compliant casing portion is formed of an elastomeric material comprising an elastic rubber or a thermoplastic elastomer.

14. The needle shield of claim 1, further comprising a rigid casing portion, wherein the rigid casing portion comprises one of:
a rigid outer casing forming at least a portion of an outer surface of the needle shield, and wherein the compliant casing portion comprises a compliant inner casing positioned within the rigid outer casing; or
a rigid core embedded within the compliant casing portion, and wherein the compliant casing portion comprises a compliant skin layer formed about the rigid core.

15. A prefilled or prefillable syringe, the syringe comprising:
a syringe assembly including:
a barrel having a barrel distal end and a barrel proximal end and defining a chamber, the barrel including a barrel end portion at the barrel distal end;
a needle having a needle tip at a distal end of the needle and having a proximal end fixedly inserted into the barrel end portion; and
the needle shield of claims **1-14,** with the needle shield mounted to the barrel via the barrel end portion, so as to be positioned over the needle; and
a plunger received within the chamber of the barrel and axially movable therein.
